# EUROPEAN PATENT SPECIFICATION

(11) **EP 2 410 871 B1**
(45) Date of publication and mention of the grant of the patent: **04.11.2015**
(21) Application number: 10719080.3
(22) Date of filing: 16.03.2010
(51) Int. Cl.: A23K 1/18, A23K 1/16, C11C 3/02

(54) **COMPOSITIONS CONTAINING C1 TO C7 ORGANIC ACID MONOGLYCERIDES AND GLYCEROL,THEIR PREPARATION AND USE AS ANTIBACTERIALS AND ANTI-MOULD AGENTS**
ZUSAMMENSETZUNGEN, DIE ORGANISCHE C1- BIS C7-SÄUREMONOGLYCERIDE UND GLYCEROL UMFASSEN, IHRE HERSTELLUNG UND IHRE VERWENDUNG ALS ANTIBAKTERIELLE MITTEL UND ANTISCHIMMELMITTEL
COMPOSITIONS CONTENANT DES MONOGLYCÉRIDES D'ACIDE ORGANIQUE EN C1 À C7 ET DU GLYCÉROL, LEUR PRÉPARATION ET LEUR UTILISATION EN TANT QU'ANTIBACTÉRIENS ET AGENTS ANTI-MOISISSURES

(30) Priority: 16.03.2009 IT FI20090050
(43) Date of publication of application: 01.02.2012
(73) Proprietor: Cantini, Fernando, 50136 Firenze (IT)
(72) Inventor: Cantini, Fernando, 50136 Firenze (IT)
(74) Representative: Brighenti, Livio
(86) International application number: PCT/IB2010/051126
(87) International publication number: WO 2010/106488

(56) References cited:
- EP-A- 1 612 260
- WO-A-2006/085346
- CA-A1- 1 050 810
- CA-A1- 1 168 078
- GB-A- 2 357 967
- SU-A1- 701 631
- US-A- 3 952 107
- P. FERREIRA ET AL.: "Esterification of glycerol with acetic acid over dodecamlybdophosphoric acid encaged in USY zeolite" CATALYSIS COMMUNICATIONS, [Online] vol. 10, 26 October 2008 (2008-10-26), pages 481-484, XP002599908 DOI: 10.1016/i.catcom.2008.10.015 Retrieved from the Internet: URL:http://dx.doi.org/10.1016/j.catcom.200 8.10.015>
- SCHUETTE H A ET AL: "SOME PHYSICAL CONSTANTS OF MONACETIN, MONOPROPIN AND MONO-NORMAL-BUTYRIN" JOURNAL OF THE AMERICAN CHEMICAL SOCIETY, AMERICAN CHEMICAL SOCIETY, WASHINGTON, DC. US, vol. 52, no. 5, 1 January 1930 (1930-01-01), pages 1978-1981, XP002516712 ISSN: 0002-7863
- M. ANTONGIOVANNI ET AL.: "Butyric acid glycerides in the diet of broiler chickens: effects on gut histolgy and carcass composition" ITALIAN JOURNAL OF ANIMAL SCIENCE, vol. 6, 2007, pages 19-25, XP002551431
- CERRATE ET AL.: "Evaluation of glycerine from biodiesel production as a feed ingredient for broilers" INTERNATIONAL JOURNAL OF POULTRY SCIENCE, [Online] vol. 5, no. 11, 2006, pages 1001-1007, XP002599909 ISSN: 1682-8356 Retrieved from the Internet: URL:http://www.pjbs.org/ijps/fin737.pdf>
- S. LEESON ET AL.: "effect of butyric acid on the performance and carcass yield of broiler chicken" POULTRY SCIENCE, vol. 84, 2005, pages 1418-1422, XP002551417
- M. ANTONGIOVANNI ET AL.: "Effect of dietary butyric glycerides on immune response and Salmonella enteriditis in broiler chicks" PROCEEDING OF 16TH EUROPEAN SYMPOSIUM ON POULTRY NUTRITION, 2007, XP002551416 Strasbourg, France & R. MAHDAVI ET AL.: "Study on usage of Period of Dietary protected butyric acid and performance,carcass characteirstics,serum metabolite levels and humoral immune response of broiler chickens" JOURNAL OF ANIMAL AND VETENARY ADVANCES, vol. 8, 2009, pages 1702-1709,
- WARREN LITSKY ET AL: "STERILITY TESTING AND ANTIMICROBIAL ACTIVITY OF COMMERCTAL GRADE GLYCERINE", INTERNET CITATION, 1 August 1971 (1971-08-01), pages 1-27, XP007919273, [retrieved on 2011-08-24]
- MARIANI E J JR ET AL: "Antimicrobial activity of commercial grade glycerine", DEVELOPMENTS IN INDUSTRIAL MICROBIOLOGY, ELSEVIER SCIENCE BV, AMSTERDAM, NL, vol. 14, 1 January 1973 (1973-01-01), pages 356-360, XP002572766, ISSN: 0070-4563

## Description

### Field of the invention

The present invention relates to the field of compositions for animal feedstuffs.

### State of the art

The most common problem known to affect farm and companion animals is the damage caused by bacterial infections and mycotoxins produced by moulds ingested with their feed. Regarding bacterial infections, and in particular intestinal infections, these often present with severe diarrhoea which can compromise, even to a serious extent, the health of the animal and consequently the revenue of the farm as a whole.

Regarding mycotoxins produced by moulds, their toxic effects on specific organs and on the physiological functions of animals, and their capacity to cause diseases such as toxicosis, have been known for some years. It is also known that carcinogenic mycotoxins, such as aflatoxins produced by moulds of the Aspergillus strain, can be transferred from cow's or goat's milk to man because they are stable and cannot be eliminated with normal heat treatments. Ocratoxin A is produced by Aspergillus and Penicillium species; if present in the feed, it can cause serious kidney diseases in pigs and in bird species.

T-2 toxin, produced by Fusarium species, can cause necrosis of the digestive tract of animals.

It is normally sought to overcome these drawbacks by using short chain organic acids, either alone or in a mixture, such as: formic acid (C₁H₂O₂), acetic acid (C₂H₄O₂), propionic acid (C₃H₅O₂), lactic acid (C₃H₆O₂), fumaric acid (C₄H₄O₄), butyric acid (C₄H₈O₂), citric acid (C₆H₈O₇) and benzoic acid (C₇H₆O₂).

Among the limitations of using said organic acids is their strong corrosive action which can damage any equipment with which they come into contact. Salified forms with ammonium, calcium, sodium or potassium also produce a corrosive action which, though more limited, is nevertheless present.

There are also certain physiological restrictions to the action of organic acids as such or in salified form when used as antibacterials in the diets of animals; in this respect organic acids are known to require an acidic pH in order to perform the actual antibacterial action, because under these conditions they are present in the undissociated RCOOH form. This undissociated RCOOH form passes through microorganism cell walls and, once inside, is dissociated according to intracellular pH. To maintain its intracellular pH the microorganism expels H⁺; the level of dissociated organic acid thus rises and as it does not leave the microorganism, it kills it.

In the intestines, however, the pH is slightly basic (around 7) so the presence of organic acid in undissociated form is very limited, and consequently the antibacterial function is also very limited.

In the light of the aforesaid, therefore, there is an evident need to develop new compositions able to counteract the effects of moulds and bacteria present in animal feeds, but which do not exhibit the aforesaid drawbacks.

The technique of microencapsulating organic acids with lipid substances was developed as a method for delivering organic acid to the intestines and to lower the pH therein, but the results obtained are rather unsatisfactory because of the buffering action of sodium bicarbonate produced by the pancreas as an intestinal pH regulator. Moreover, in addition to not being very effective, this technology is also very costly.

The antibacterial action of certain fatty acid monoglycerides has been investigated in a number of studies, for example:
1. J. Kabara, Dennis M. Swieczkowski, Anthony J, Conley, Joseph P. Truant 1972 FATTY ACIDS AND DERIVATES AS ANTIMICROBIAL AGENTS.
2. G. Bergsson, J. Arnfinnsson S. Karlsson, O. Steingrimsson, H. Thormar 1998. IN VITRO INACTIVATION OF CHLAMYDIA TRACHOMATIS BY FATTY ACIDS AND MONOGLYCERIDES.
3. G. Bergsson, J. Arnfinnsson, O. Steingrimsson, H. Thormar 2001. IN VITRO KILLING OF CANDIDA ALBICANS BY FATTY ACIDS AND MONOGLYCERIDES.
4. H. Thormar, H. Hilmarsson, G. Bergsson 2005. STABLE CONCENTRATED EMULSIONS OF THE 1-MONOGLYCERIDE OF CAPRIC ACID (MONOCAPRIN) WITH MICROBICIDAL ACTIVITIES AGAINST THE FOOD-BORNE BACTERIA CAMPYLOBACTER JEJUNI, SALMONELLA SPP AND ESHERICHIA COLI; PCT/IS2005/000026 "STABLE CONCENTRATED ANTI-BACTERIAL EMULSIONS OF MONOCAPRIN IN WATER".
5. Hilmarsson, H. Thormar, J.H. Thrainsson, E. Gunnarsson 2006 EFFECT OF GLYCEROL MONOCAPRATE (MONOCAPRIN) ON BROILER CHICKENS: AN ATTEMPT AT REDUCING INTESTINAL CAMPYLOBACTER INFECTION.

These studies have highlighted a promising but not exhaustive research direction. SU 701 631 describes the high bactericidal power of monoglyceride of propionic acids and its suppressing activity on fungi.

Mauro Antongiovanni et al. " Effect of dietary butyric glycerides on immune response and Salmonella enteriditis in broiler chicks" in Proceedings of 16th European Symposium on poultry nutrition, 2007, XP002551416 Strasburg, France reports the immune modulation effect of butyric glycerides on serum immunoglobulin and on *Salmonella enteriditis* colonization; the neat effect of butyric glycerides in controlling the infection of *Salmonella* is described.

Mauro Antongiovanni et al. "Butyric Acid glycerides in the diet of broiler chickens: effects on gut histology and carcass composition" in Ital. J. Anim. sci. Vol. 6, 19-25 (2007) describes the effects of butyric acids glycerides as a supplemental ingredient in the diet on live performance of broiler chickens and on the morphology of their small intestine.

CA 1 168 078 describes that Monobutyrin and Monoproprionine can be used in intermediate-moisture food compositions possibly with usual preservative to prevent the growth of bacteria and moulds; there is no mention of a pre-mixing of monobutyrin or monoproprionine with glycerol before application on the products to be treated.

As far as current knowledge allows, there are no studies which confirm the specifically antibacterial and anti-mould action of compositions of short chain fatty acid monoglycerides combined with glycerol.

### Brief description of the figures

Figure 1 is an aqueous suspension of a composition of the invention shown by electron microscopy.

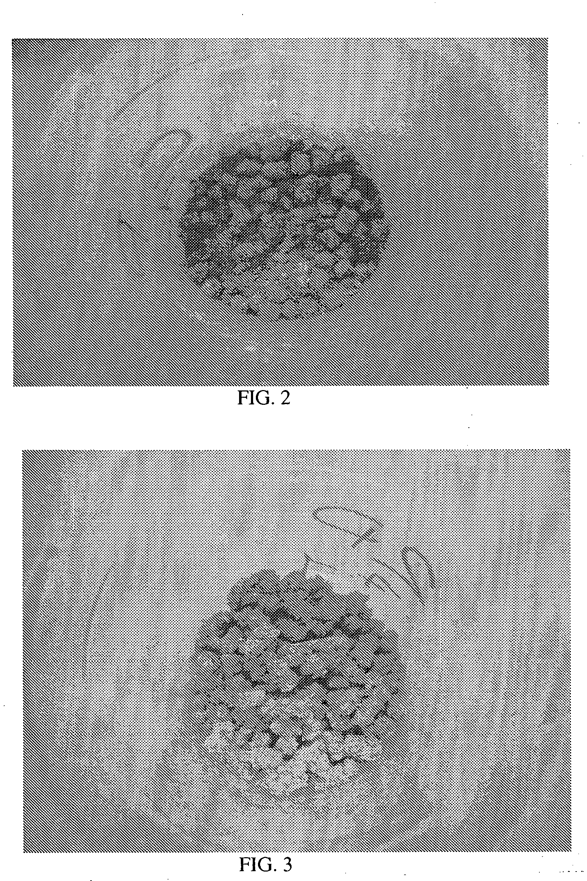

Figure 2 shows untreated feed inoculated with Fusarium.
Figure 3 shows feed inoculated with Fusarium and treated with 0.7% Monopropionin 43.

### Summary of the invention

The present invention relates to compositions containing C₁ to C₇ fatty acid monoglycerides in percentages between 10% and 90% and glycerol between 10 and 90% by weight (calculated on the total composition weight) as antibacterials and anti-mould agents to be added to cereals, feed, and to general foodstuffs and drinking water intended for the feeding of animals.

### Detailed description of the invention

It has surprisingly been found that compositions containing C₁ to C₇ organic acid monoglyceride esters combined with glycerol have a strong antibacterial potency both at acidic pH (4.5) and at neutral pH as is present in animal intestines (i.e. pH 7).

In the compositions of the invention, the organic acid monoglyceride esters as aforedefined are present in amounts between 10% and 90% and the glycerol between 10 and 90% by weight (calculated on the total composition weight); preferably said amounts are between 40%-90%, and 10%-60% respectively.

The term "C₁ to C₇ organic acids" according to the invention refers preferably to the following acids: formic, acetic, propionic, lactic, butyric, citric, fumaric and benzoic acids.

Butyric acid and propionic acid are particularly preferred.

Examples of compositions according to the invention are compositions consisting of:
(a)

| | |
|---|---|
| monoglyceride ester of butyric acid | 42 - 47% |
| diglyceride ester of butyric acid | 5 - 8% |
| triglyceride ester of butyric acid | 0.5 - 2% |
| glycerol | 45 - 50% |

(b)

| | |
|---|---|
| monoglyceride ester of propionic acid | 45-50% |
| diglyceride ester of propionic acid | 8-12% |
| triglyceride ester of propionic acid | 1-3% |
| glycerol | 36-40% |

Specific examples of compositions according to the invention are compositions consisting of:
(c)

| | |
|---|---|
| monoglyceride ester of butyric acid | 45% |
| diglyceride ester of butyric acid | 6% |
| triglyceride ester of butyric acid | 1% |
| glycerol | 48% |

(d)

| | |
|---|---|
| butyric acid monoglycerides | 43% |
| butyric acid diglycerides | 6% |
| butyric acid triglycerides | 1% |
| glycerol | 50% |

(e)

| | |
|---|---|
| monoglyceride ester of propionic acid | 49% |
| diglyceride ester of propionic acid | 10% |
| triglyceride ester of propionic acid | 2% |
| glycerol | 39% |

(f)

| | |
|---|---|
| propionic acid monoglycerides | 43% |
| propionic acid diglycerides | 6% |
| propionic acid triglycerides | 1% |
| glycerol | 50% |

Antibacterial potency values of organic acids alone compared with those of the compositions of the invention are given below in table 1.

**Table 1**

| PRODUCT | CONC. USED | pH | E.coli (cfu/ml) | Salmonella typhimurium (cfu/ml) | Campylobacter jejuni (cfu/ml) |
|---|---|---|---|---|---|
| Positive control | | 7 | 108X10⁵ | 120X10⁵ | 431X10⁵ |
| Positive control | | 4.5 | 34X10⁵ | 96X10⁵ | 201X10⁵ |
| Propionic acid | 1:909 | 7 | 54X10⁴ | 11X10⁴ | 33X10³ |
| Propionic acid | 1:909 | 4.5 | 13X10⁴ | 42X10³ | 14X10¹ |
| Butyric Acid | 1:1000 | 7 | 106X10⁴ | 65X10⁴ | 18X10³ |
| Butyric Acid | 1:1000 | 4.5 | 78X10⁴ | 25X10³ | 2X10¹ |
| Monopropionin 43 | 1:109 | 7 | 110X10⁴ | 12X10³ | 79X10⁵ |
| Monobutyrin 43 | 1:100 | 7 | 75X10⁴ | 35X10³ | 48X10⁴ |
| Monobutyrin 43 | 1:100 | 45 | 47X10⁴ | 8X10² | 87X10³ |

| | | | | | |
|---|---|---|---|---|---|
| Note: Monopropionin 43 is composed of: 43% propionic acid monoglycerides 6% propionic acid diglycerides 1% propionic acid triglycerides 50% glycerol Note: Monobutyrin 43 is composed of: 43% butyric acid monoglycerides 6% butyric acid diglycerides 1 % butyric acid triglycerides 50% glycerol | | | | | |

Table 2 compares the in vitro antibacterial action of pure butyric acid, of butyric acid monoglycerides without free glycerol and of a mixture of butyric acid monoglycerides with free glycerol, against Clostridium perfringens. Whereas the mixture of butyric acid monoglycerides and glycerol already exhibits an inhibitory potency (i.e. no growth) in all three replicates at a concentration of 1000 ppm, the butyric acid monoglycerides do not exhibit any inhibitory potency against the bacterium, and butyric acid only exhibits inhibitory action from 3000 ppm.

**TABLE 2**

| Bacteria: Clostridium perfringens CP27 | | | | | |
|---|---|---|---|---|---|
| Inoculation concentration: 10⁵ | | | | | |
| Medium: Brain Heart Infusion | | | | | |
| Incubation time and appearance of growth; + for 24hr, ++ for 37hr and +++ for 96hr - 3 replications for each concentration | | | | | |
| Negative | Positive | | | | |
| Control | control (PC) | ppm | Butyric Acid | Monobutyrin 43 | Butyric acid monoglycerides |
| | + | 500 | + | + | + |
| | + | | + | + | + |
| | ++ | | + | + | + |
| | | mean | | | |
| | | 1000 | + | no growth | + |
| | | | + | no growth | + |
| | | | + | no growth | + |
| | | mean | | | |
| | | 1500 | + | no growth | + |
| | | | + | no growth | + |
| | | | + | no growth | + |
| | | mean | | | |
| | 2000 | 2000 | ++ | no growth | + |
| | | | ++ | no growth | + |
| | | ++ | ++ | no growth | + |
| | | mean | | | |
| | | 2500 | ++ | no growth | + |
| | | | ++ | no growth | + |
| | | | ++ | no growth | + |
| | | mean | | | |
| | | 3000 | no growth | no growth | + |
| | | | no growth | no growth | + |
| | | | no growth | no growth | + |
| | | mean | | | |
| | 4000 | 4000 | no growth | no growth | + |
| | | | no growth | no growth | + |
| | | | no growth | no growth | + |
| | | mean | | | |

Table 3 compares the in vitro antibacterial action of pure acetic acid, of acetic acid monoglycerides without free glycerol and of a mixture of acetic acid monoglycerides with free glycerol against porcine *Salmonella typhimurium.* Whereas the mixture of acetic acid monoglycerides and glycerol (Monoacetin 42) exhibits an inhibitory potency (i.e. no growth) in all three replicates at a concentration of 10,000 ppm, the acetic acid monoglycerides exhibit inhibitory potency against the bacterium from 25,000 ppm and the acetic acid exhibits inhibitory action from 20,000 ppm.

**TABLE 3**

| Bacteria: Porcine *Salmonella typhimurium* | | | | | |
|---|---|---|---|---|---|
| Inoculation concentration: 10⁵ | | | | | |
| Medium: Brain Heart Infusion | | | | | |
| Incubation time and appearance of growth: + for 24hr, ++ for 37hr and +++ for 96hr - 3 replications for each concentration | | | | | |
| pH 6 | | | | | |
| Negative | Positive | | | | |
| control | Control (PC) | ppm | Acetic acid | Monoacetin 42 | Acetic acid monoglycerides |
| | + | 5000 | + | + | + |
| | + | | + | + | + |
| | ++ | | + | + | + |
| | | Mean | | | |
| | | 10000 | + | no growth | + |
| | | | + | no growth | + |
| | | | + | no growth | + |
| | | Mean | | | |
| | | 15000 | + | no growth | + |
| | | | + | no growth | + |
| | | | + | no growth | + |
| | | Mean | | | |
| | | 20000 | no growth | no growth | + |
| | | no growth | no growth | no growth | + |
| | | | no growth | no growth | + |
| | | Mean | | | |
| | | 25000 | no growth | no growth | no growth |
| | | | no growth | no growth | no growth |
| | | | no growth | no growth | no growth |
| | | Mean | | | |
| | | 30000 | no growth | no growth | no growth |
| | | | no growth | no growth | no growth |
| | | | no growth | no growth | no growth |
| | | Mean | | | |
| | | 40000 | no growth | no growth | no growth |
| | | | no growth | no growth | no growth |
| | | | no growth | no growth | no growth |
| | | Mean | | | |

| | | | | | |
|---|---|---|---|---|---|
| Note: Monoacetin 42 is composed of: 42% acetic acid monoglycerides 7% acetic acid diglycerides 1 % acetic acid triglycerides 50% glycerol | | | | | |

Table 4 compares the in vitro antibacterial action of pure formic acid, of formic acid monoglycerides without free glycerol and of a mixture of formic acid monoglycerides with free glycerol against porcine *Salmonella typhimurium.* Whereas the mixture of formic acid monoglycerides and glycerol (Monoformin 42) exhibits an inhibitory potency (i.e. no growth) in all three replicates at a concentration of 5,000 ppm, the formic acid monoglycerides exhibit inhibitory potency against the bacterium from 25,000 ppm and formic acid exhibits inhibitory action from 15,000 ppm.

**TABLE 4**

| Bacteria: Porcine *Salmonella typhimurium* | | | | | |
|---|---|---|---|---|---|
| Inoculation concentration: 10⁵ | | | | | |
| Medium: Brain Heart Infusion | | | | | |
| Incubation time and appearance of growth: + for 24hr, ++ for 37hr and +++ for 96hr - 3 replications for each concentration | | | | | |
| pH 6 | | | | | |
| Negative | Positive | | | | |
| control | Control (PC) | ppm | Formic acid | Monoformin 42 | Formic acid monoglycerides |
| + | + | 5000 | + | no growth | + |
| + | + | | + | no growth | + |
| | ++ | | + | no growth | + |
| | | Mean | | | |
| | | 10000 | + | no growth | + |
| | | | + | no growth | + |
| | | | + | no growth | + |
| | | Mean | | | |
| | | 15000 | no growth | no growth | + |
| | | | no growth | no growth | + |
| | | | no growth | no growth | + |
| | | Mean | | | |
| | 20000 | 20000 | no growth | no growth | + |
| | | | no growth | no growth | + |
| | | | no growth | no growth | + |
| | | Mean | | | |
| | | 25000 | no growth | no growth | no growth |
| | | | no growth | no growth | no growth |
| | | | no growth | no growth | no growth |
| | | Mean | | | |
| | | 30000 | no growth | no growth | no growth |
| | | | no growth | no growth | no growth |
| | | | no growth | no growth | no growth |
| | | Mean | | | |
| | | 40000 | no growth | no growth | no growth |
| | | | no growth | no growth | no growth |
| | | | no growth | no growth | no growth |
| | | Mean | | | |

| | | | | | |
|---|---|---|---|---|---|
| Note: Monoformin 42 is composed of: 42% formic acid monoglycerides 7% formic acid diglycerides 1% formic acid triglycerides 50% glycerol | | | | | |

Table 5 compares the in vitro antibacterial action of pure fumaric acid, of fumaric acid monoglycerides without free glycerol and of a mixture of fumaric acid monoglycerides with free glycerol (Monofumarin 41) against E. *coli.* Whereas the mixture of fumaric acid monoglycerides and glycerol exhibits an inhibitory potency (i.e. no growth) in all three replicates at a concentration of 20,000 ppm, the fumaric acid monoglycerides exhibit inhibitory potency against the bacterium from 60,000 ppm and the fumaric acid exhibits inhibitory action from 90,000 ppm.

**TABLE 5**

| Bacteria: *E. coli* | | | | | |
|---|---|---|---|---|---|
| Inoculation concentration: 10⁵ | | | | | |
| Medium: Brain Heart Infusion | | | | | |
| Incubation time and appearance of growth: + for 24hr, ++ for 37hr and +++ for 96hr - 3 replications for each concentration | | | | | |
| pH 5 | | | | | |
| Negative | Positive | | | | |
| control | Control (PC) | Ppm | Fumaric acid | Monofumarin 41 | Fumaric acid monoglycerides |
| | + | 10000 | + | + | + |
| | + | | + | + | + |
| | ++ | | + | + | + |
| | | Mean | | | |
| | 20000 | 20000 | + | no growth | + |
| | | | + | no growth | + |
| | | | + | no growth | + |
| | | Mean | | | |
| | | 40000 | + | no growth | + |
| | | | + | no growth | + |
| | | | + | no growth | + |
| | | Mean | | | |
| | | 60000 | ++ | no growth | no growth |
| | | | ++ | no growth | no growth |
| | | | ++ | no growth | no growth |
| | | Mean | | | |
| | 80000 | 80000 | ++ | no growth | no growth |
| | | | ++ | no growth | no growth |
| | | | ++ | no growth | no growth |
| | | Mean | | | |
| | | 90000 | no growth | no growth | no growth |
| | | | no growth | no growth | no growth |
| | | | no growth | no growth | no growth |
| | | Mean | | | |
| | | 100000 | no growth | no growth | no growth |
| | | | no growth | no growth | no growth |
| | | | no growth | no growth | no growth |
| | | Mean | | | |

| | | | | | |
|---|---|---|---|---|---|
| Nota: Monofumarin 41 is composed of: 41% fumaric acid monoglycerides 8% fumaric acid diglycerides 1% fumaric acid triglycerides 50% glycerol | | | | | |

If preferred, the compositions of the invention can also contain active principles of essential oils (cinnamic aldehyde, thymol, carvacrol) in percentages comprised between 1 and 20% (calculated by weight on the weight of the mixture of other components) as commonly provided for such products for feeding animals, since these active principles are soluble in lipids but insoluble in glycerol.

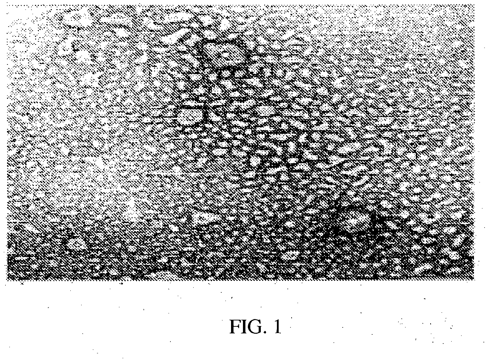

It should be noted that when the composition of the invention is dispersed in water, the glycerol surrounds the monoglyceride itself to form drops which incorporate said monoglyceride, they remaining suspended in water (the other optionally added active principles dissolve in the monoglyceride, they also becoming incorporated within the glycerol drop) (see fig.1).

The compositions of the invention can be prepared according to the usual fatty acid esterification processes amply described in the literature, but using a large excess of glycerol (never less than 200% by weight on the weight of the fatty acids used) in order to obtain a large amount of monoglycerides with large amounts of free glycerol.

The compositions of the invention can be added to the animal feed and/or their drinking water in amounts from 0.1 to 1.5%, preferably from 0.3-0.6% calculated by weight on the feed or drink weight.

The compositions of the invention are particularly indicated for the diets of pigs, chickens, fish, cattle, sheep and companion animals.

### EXAMPLE 1

The esterification reaction takes place in batches of 10,000 kg.

3000 kg of butyric acid and 7000 kg of glycerol are introduced into a reactor at ambient temperature.

The temperature is increased to 140°C, the butyric acid that evaporates being recycled within the reactor by means of a reflux condenser.

The further raising of the temperature from 140 to 170°C must be very slow (over about 4 hours) and the reflux condenser temperature must be maintained at 120°C in order to evaporate the water derived from the esterification reaction while the butyric acid continues to recycle within the reactor.

At this point the temperature can be raised to 180°C (but leaving the reflux condenser temperature at 120°C) and once this temperature has been reached the acidity of the mixture is expected to reach a value less than 1%.

A vacuum is then applied to distil off any unreacted butyric acid until a final acidity of less than 0.2% is reached.

The mixture is discharged through a cooler to bring it to ambient temperature.

A mixture is thus obtained containing 43% monoglyceride ester, 6% diglyceride ester, 1% triglyceride ester, and 50% glycerol.

Once the esterification reaction is complete the glycerol can be separated if desired by distillation from the thus obtained mono- di- and triglyceride esters to arrive at a 90% monoglyceride concentration.

### EXAMPLE 2

Sixty 5 week old DanBred piglets were assigned to two groups of thirty piglets each: A) - control, and B) treated, divided into 6 pens of ten animals each. After the first 10 days of adaptation in the enclosures, all animals were inoculated orally with Salmonella typhimurium, isolated at the Istituto Zooprofilattico of Forlì (Italy) from fecal samples of infected pigs, with a dose equal to 7 x 10⁷ cfu.

The following day some of the subjects from each pen presented with diarrhoea. The symptoms worsened and affected all the subjects over the next three days following infection.

Fecal samples were collected on the third day following Infection; the bacterial count was found to be equal to 165,000 cfu in control group A) and 1,60,000 cfu in the treated group B). Group B) from the third day after infection was treated with a mixture composed of:
- Butyric acid monoglycerides = 45%
- Butyric acid diglycerides = 6%
- Butyric acid triglycerides = 1%
- Glycerol = 48%
administered in the drinking water at a dosage of 0.5% for three days. On the third day after treatment, fecal samples were again collected for bacterial count analysis. The control group A) presented a mean cfu number of 160,000, while in the treated group B) the cfu number was 900. Use of the "butyric acid esters and glycerol" mixture in the stated percentages reduced the cfus of salmonella by 3 log10, with a 3-day administration. This fact confirms the bactericidal effectiveness of the mixture.

### EXAMPLE 3

The present field trial was carried out on an Italian farm with hygiene problems such as very evident ileitis resulting from a Lawsonia intracellularis infection, enteritis from Brachyspira Spp and necrotic enteritis resulting from a Treponema hyodysenteriae infection. 1,027 DanBred pigs weighing about 25 kg (71 days old) were divided into two groups: control group A) and treated group B), composed of 511 and 516 animals respectively.

The two groups were fed with a feed that was formulated in identical manner except for the following components: the feed of the control group had added Lincomycin, 200 ppm, and Doxicyclin, 250 ppm, for the first 14 days of the trial, and Lincomycin alone for the remaining time. The treated group B) did not receive antibiotics in the feed, only a "butyric acid esters and glycerol" mixture composed as follows:
- Butyric acid monoglycerides = 45%
- Butyric acid diglycerides = 6%
- Butyric acid triglycerides = 1 %
- Glycerol = 48%
administered to the feed in a quantity of 0.5% to replace 0.5% of the soya oil. The trial lasted 63 days. The growth and feeding efficiency results are summarized in the table below.

**Table**

| | Group A) - Control | Group B) - Butyric acid esters and glycerol | Delta |
|---|---|---|---|
| No. of animals | 511 | 516 | |
| Age at the start of the trial (days) | | 71 | |
| Age at the end of the trial (days) | 134 | 134 | |
| Average weight at the start of the trial (kg) | 25 | 25 | |
| Average weight at the end of the trial (kg) | 62.13 | 63.61 | |
| No. of dead animals | 5 | 2 | -3 |
| No. of rejected animals | 3 | 2 | -1 |
| Average daily weight increase (kg) | 0.59 | 0.61 | + 0.02 |
| Total feed consumed (kg) | 53.570 | 53.250 | -320 |
| Meat produced in kg | 19.340 | 20.200 | +860 |
| Feed conversion index | 2.76 | 2.64 | -0.12 |

Although the fecal analysis of the control group A) showed the presence of Lawsonia, its presence was not found in the treated group B). The diarrhoea episodes were also very much reduced in the treated group B). The growth parameters, the feed conversion index of the treated group B) were comparable, and tendentially better than those of the control group A) whose diet contained the aforesaid antibiotics. The "butyric acid esters and glycerol" mixture enabled the highlighted diseases to be controlled, without the use of antibiotics. The trial has demonstrated the antibacterial effect of the "butyric acid esters and glycerol" mixture with a consequent improvement to intestinal health.

### EXAMPLE 4

### Efficacy test towards Salmonella typhimurium in chickens

### Salmonella strain

For the test, a strain of Salmonella typhimurium isolated and identified by the IZSLER section of Forlì was used.

### Animals

SPF (Specific Pathogen Free) chicks were used, 30 animals per test. The chicks were hatched at the IZSLER section of Forlì. The subjects were immediately placed into isolation units.

### Diet

The animals received water from the mains water supply and a commercial starter ad libitum feed. The feed contained added Monobutyrin 43.

### Experimental protocol

4 groups of 30 subjects each were prepared. The diets differed by the different amount of Monobutyrin 43 added to the feed from the first day of life, and were identified as follows: untreated control group: 0%, group 1: 1% in the feed, group 2: 0.3% in the feed. Group 3 received the same feed as the control group up to the 14^{th} day of life, i.e. until the 7^{th} day post-infection, and only received feed supplemented with 1.4% Monobutyrin 43 after that day.

At aged 7 days, all the subjects were infected by the esophageal route with 10⁷ cfu of Salmonella typhimurium. 24 hours following infection, cloacal swabs were taken from all the subjects to confirm that Salmonella typhimurium infection had taken hold. At 14, 24 and 35 days of life, 10 subjects in each group were killed. The ceca were collected from each animal and the load of Salmonella typhimurium was determined (expressed in cfu/g).

### Laboratory tests

The absence of antibodies against S. typhimurium was confirmed by an ELISA test. The cloacal swabs were seeded directly onto Hektoen Enteric Agar and incubated at 37°C for 24 hours. One gram of intestinal contents was diluted in 9 ml of Ringer's lactate and seeded onto Hektoen Enteric Agar (inoculum volume: 0.1 ml). Colony counting was carried out after 24 hours of incubation at 37°C. For each collection, the geometric means of the bacterial loads of the 10 killed subjects were calculated.

All the subjects, after one day of life, were found to be seronegative for Salmonella typhimurium. 24 hours after the infection, all the cloacal swabs were found to be positive for S. typhimurium. The results of the determined cecal bacterial loads are shown in the following table.

**Table**

| CFU in the cecum of chickens infected with Salmonella Typhimurium - 10⁷ | | | | |
|---|---|---|---|---|
| | Control | Group 1 (1% in feed) | Group 2 (0.3% in feed) | Group 3 (1.4% in feed from 14^{th} day of life) |
| Day of infection (7^{th} day of life) | 0 | 0 | 0 | 0 |
| 7^{th} day post-infection | 6,400,000 | 770,000 | 2,226,000 | 6,302,000 Start of treatment |
| 17^{th} day post-infection | 25,120,000 | 213,220 | 1,242,000 | 171,120 |
| 28^{th} day post-infection | (high mortality) | >100 | 300 | 1,000 |

### EXAMPLE 5

### In vitro sensitivity tests towards filamentous fungi (moulds)

### Materials and methods

Strains of Aspergillus spp, Penicillium spp and Fusarium spp were utilized for the test, having been isolated and identified during diagnostic activity at the IZSLER section of Forlì from complete feeds used in the chicken industry. To prepare the inoculum, mycelium of pure cultures of the tested strains was collected using a swab. The material thus collected was dissolved in a culture broth (BHI - Brain Heart Infusion). 5 ml of the fungal suspension and an equal amount of the product to be tested were placed in contact in a test tube. The test tube was incubated at 20±4°C for 24 hours.

After this time period, the fungal suspension was then seeded and enumerated. The control suspension was obtained by placing 5 ml of fungal suspension + 5 ml of diluent (Ringer's lactate) into a test tube. Reading of the tests was carried out after a 5 day incubation period at 20 ± 4°C.

The results given in the following table are expressed as cfu/ml

**Table**

| PRODUCT | ASPERGILLUS spp. | PENICILLIUM spp. | FUSARIUM spp: |
|---|---|---|---|
| Control | 450000 | 97000 | 310000 |
| Monopropionin 43 | 20000 | 1500 | 90000 |
| Monobutyrin 43 | 1000 | 700 | 3000 |
| Propionic acid | 300 | <100 | 300 |
| Ammonium propionate | 1000 | 100 | 500 |

| | | | |
|---|---|---|---|
| Note: Monopropionin 43 is composed of: 43% propionic acid monoglycerides 12% propionic acid diglycerides 1 % propionic acid triglycerides 28% free glycerol 16% H₂O Note: Monobutyrin 43 is composed of: 43% butyric acid monoglycerides 6% butyric acid diglycerides 1 % butyric acid triglycerides 50% glycerol | | | |

### EXAMPLE 6

### In vitro efficacy test towards Penicilium spp and Fusarium spp

### Materials and methods

Strains: strains of moulds isolated and identified by the IZSLER section at Forlì were used for the test. The strains were revitalized in BHI broth then enumerated in OGYE agar (after incubation at 20°C for 5 days)

Substrate: a complete chicken feed, sterilized in a dry oven at 100°C for 4 hours, was used.

Efficacy test: 10g of feed were inoculated with 2 ml of fungal suspension (in distilled water) to which 70 µl of the product to be tested was added. The mixture thus obtained was kept at ambient temperature. A positive control (infected and untreated) and a negative control (feed only + distilled water) were also prepared. On days 7 and 14 following infection, the fungal concentrations in the treated sample and control samples were evaluated.

The results are given in the table below.

**TABLE**

| PRODUCT | Concentration of Fusarium spp. On day 0 (cfu/g) | Concentration of Fusarium spp. 7 days post-infection (cfu/g) | Concentration of Fusarium spp. 14 days post-infection (cfu/g) | Concentration of Penicillium spp. On day 0 (cfu/g) | Concentration of Penicillium spp. 7 days post-infection (cfu/g) | Concentration of Penicillium spp. 14 days post-Infection (cfu/g) |
|---|---|---|---|---|---|---|
| Positive | 5,700,000 | 72,000,000 | 300,000,000 | 100,000 | 30,000,000 | 200,000,000 |
| control | | | | | | |
| Negative control | <100 | <100 | <100 | <100 | <100 | <100 |
| Monopropi onin 43 | 5,700,000 | 410,000 | 250,000 | 100,000 | <100 | <100 |

| | | | | | | |
|---|---|---|---|---|---|---|
| Note: Monopropionin 43 is composed of: 43% propionic acid monoglycerides 12% propionic acid diglycerides 1 % propionic acid triglycerides 28% free glycerol 16% H₂O | | | | | | |

## Claims

1. Compositions containing C₁ to C₇ organic acid monoglycerides in percentages between 10% and 90% by weight calculated on the total composition weight for use as antibacterial and antimould compositions for treating animals **characterised in that** such composition contains glycerol in percentages between 10% and 90% by weight calculated on the total composition weight.

2. Compositions according to claim 1 wherein said compositions contain C₁ to C₇ organic acid monoglycerides in percentages between 40%-90% and glycerol in percentages between 10%-60% by weight calculated on the total composition weight.

3. Compositions according to claims 1 and 2 wherein said organic acids are chosen from: formic, acetic, propionic, lactic, butyric, citric, fumaric and benzoic acids.

4. Compositions according to claim 3 wherein said acids are butyric acid and propionic acid.

5. Compositions according to claims 1-4 consisting of:
(a)
| | |
|---|---|
| monoglyceride ester of butyric acid | 42 - 47% |
| diglyceride ester of butyric acid | 5 - 8% |
| triglyceride ester of butyric acid | 0.5 - 2% |
| glycerol | 45 - 50% |
or
(b)
| | |
|---|---|
| monoglyceride ester of propionic acid | 45-50% |
| diglyceride ester of propionic acid | 8-12% |
| triglyceride ester of propionic acid | 1-3% |
| glycerol | 36-40% |
or
(c)
| | |
|---|---|
| monoglyceride ester of butyric acid | 45% |
| diglyceride ester of butyric acid | 6% |
| triglyceride ester of butyric acid | 1% |
| glycerol | 48% |
or
(d)
| | |
|---|---|
| butyric acid monoglycerides | 43% |
| butyric acid diglycerides | 6% |
| butyric acid triglycerides | 1% |
| glycerol | 50% |
or
(e)
| | |
|---|---|
| monoglyceride ester of propionic acid | 49% |
| diglyceride ester of propionic acid | 10% |
| triglyceride ester of propionic acid | 2% |
| glycerol | 39% |
or
(f)
| | |
|---|---|
| propionic acid monoglycerides | 43% |
| propionic acid diglycerides | 6% |
| propionic acid triglycerides | 1% |
| glycerol | 50% |
wherein said percentages are expressed by weight

6. Compositions according to claims 1-5 also containing active principles of essential oils in percentages comprised between 1-20% (by weight).

7. Use of compositions according to claims 1-6 as antibacterial and anti-mould compositions for treating cereals, feeds and general foodstuffs and drinking water intended for feeding animals.

8. Animal feeds or drinks, either liquid or solid, comprising a composition containing C₁ to C₇ organic acid monoglycerides in percentages between 10% and 90% by weight calculated on the total composition weight for use as antibacterial and antimould compositions for treating animals **characterised in that** such composition contains glycerol in percentages between 10% and 90% by weight calculated on the total composition weight.

9. Feeds or drinks according to claim 8 wherein said compositions are added in an amount between 0.1 and 1.5%, preferably between 0.3 and 0.5% by weight on the total weight of the feed or drink.

10. Feeds according to claims 8 and 9 wherein said feeds are those used for the feeding of pigs, chickens, fish, cattle, sheep and companion animals.

## Patentansprüche

1. Zusammensetzungen, die Monoglyceride organischer C₁- bis C₇-Säuren in Prozentsätzen zwischen 10 Gew.-% und 90 Gew.-%, berechnet anhand des Gesamtzusammensetzungsgewichts, zur Verwendung als antibakterielle Zusammensetzungen und Schimmelverhütungszusammensetzungen zur Behandlung von Tieren enthalten, **dadurch gekennzeichnet, dass** diese Zusammensetzungen Glycerin in Prozentsätzen zwischen 10 Gew.-% und 90 Gew.-%, berechnet anhand des Gesamtzusammensetzungsgewichts, enthalten.

2. Zusammensetzungen gemäß Anspruch 1, wobei die genannten Zusammensetzungen Monoglyceride organischer C₁- bis C₇-Säuren in Prozentsätzen zwischen 40 Gew.-% - 90 Gew.-% und Glycerin in Prozentsätzen zwischen 10 Gew.-% - 60 Gew.-%, berechnet anhand des Gesamtzusammensetzungsgewichts, enthalten.

3. Zusammensetzungen gemäß Anspruch 1 und 2, wobei die genannten organischen Säuren gewählt sind aus: Ameisensäure, Essigsäure, Propionsäure, Milchsäure, Buttersäure, Zitronensäure, Fumarsäure und Benzoesäure.

4. Zusammensetzungen gemäß Anspruch 3, wobei die genannten Säuren Buttersäure und Propionsäure sind.

5. Zusammensetzungen gemäß den Ansprüchen 1-4, bestehend aus:
(a)
| | |
|---|---|
| Monoglyceridester von Buttersäure | 42 - 47 % |
| Diglyceridester von Buttersäure | 5 - 8 % |
| Triglyceridester von Buttersäure | 0,5 - 2 % |
| Glycerin | 45 - 50 % |
oder
(b)
| | |
|---|---|
| Monoglyceridester von Propionsäure | 45 - 50 % |
| Diglyceridester von Propionsäure | 8 - 12 % |
| Triglyceridester von Propionsäure | 1 - 3 % |
| Glycerin | 36 - 40 % |
oder
(c)
| | |
|---|---|
| Monoglyceridester von Buttersäure | 45 % |
| Diglyceridester von Buttersäure | 6 % |
| Triglyceridester von Buttersäure | 1 % |
| Glycerin | 48 % |
oder
(d)
| | |
|---|---|
| Buttersäuremonoglyceride | 43 % |
| Buttersäurediglyceride | 6 % |
| Buttersäuretriglyceride | 1 % |
| Glycerin | 50 % |
oder
(e)
| | |
|---|---|
| Monoglyceridester von Propionsäure | 49 % |
| Diglyceridester von Propionsäure | 10 % |
| Triglyceridester von Propionsäure | 2 % |
| Glycerin | 39 % |
oder
(f)
| | |
|---|---|
| Propionsäuremonoglyceride | 43 % |
| Propionsäurediglyceride | 6 % |
| Propionsäuretriglyceride | 1 % |
| Glycerin | 50 % |
wobei die genannten Prozentsätze in Gewichtsprozent ausgedrückt sind.

6. Zusammensetzungen gemäß den Ansprüchen 1-5, die außerdem aktive Grundbestandteile etherischer Öle in Prozentsätzen zwischen 1-20 (Gew.-)% enthalten.

7. Verwendung von Zusammensetzungen gemäß den Ansprüchen 1-6 als antibakterielle Zusammensetzungen und Schimmelverhütungszusammensetzungen zur Behandlung von Getreiden, Futter und allgemeinen Futtermitteln und Trinkwasser, die zur Fütterung von Tieren bestimmt sind.

8. Tierfutter oder -getränke, entweder flüssig oder fest, die eine Zusammensetzung umfassen, die Monoglyceride organischer C₁- bis C₇-Säuren in Prozentsätzen zwischen 10 Gew.-% und 90 Gew.-%, berechnet anhand des Gesamtzusammensetzungsgewichts, zur Verwendung als antibakterielle Zusammensetzungen und Schimmelverhütungszusammensetzungen zur Behandlung von Tieren enthält, **dadurch gekennzeichnet, dass** diese Zusammensetzung Glycerin in Prozentsätzen zwischen 10 Gew.-% und 90 Gew.-%, berechnet anhand des Gesamtzusammensetzungsgewichts, enthält.

9. Futter oder Getränke gemäß Anspruch 8, wobei die genannten Zusammensetzungen in einer Menge zwischen 0,1 und 1,5 Gew.-%, vorzugsweise zwischen 0,3 und 0,5 Gew.-%, anhand des Gesamtgewichts des Futters oder Getränks, zugegeben werden.

10. Futter gemäß den Ansprüchen 8 und 9, wobei die genannten Futter jene sind, die zum Füttern von Schweinen, Hühnern, Fischen, Vieh, Schafen und Haustieren verwendet werden.

## Revendications

1. Compositions contenant des monoglycérides d'acides organiques en C₁ à C₇ dans des pourcentages situés entre 10 % et 90 % en poids calculés sur le poids total de la composition pour une utilisation en tant que compositions antibactériennes et anti-moisissures pour le traitement d'animaux **caractérisées en ce qu'**une telle composition contient du glycérol dans des pourcentages situés entre 10 % et 90 % en poids calculés sur le poids total de la composition.

2. Compositions selon la revendication 1, lesdites compositions contenant des monoglycérides d'acides organiques en C₁ à C₇ dans des pourcentages situés entre 40 % et 90 % et du glycérol dans des pourcentages situés entre 10 % et 60 % en poids calculés sur le poids total de la composition.

3. Compositions selon les revendications 1 et 2, dans lesquelles lesdits acides organiques sont choisis parmi : les acides formique, acétique, propionique, lactique, butyrique, citrique, fumarique et benzoïque.

4. Compositions selon la revendication 3, dans lesquelles lesdits acides sont l'acide butyrique et l'acide propionique.

5. Compositions selon les revendications 1 à 4 constituées de :
(a)
ester monoglycéride de l'acide butyrique - 42 à 47 %
ester diglycéride de l'acide butyrique - 5 à 8 %
ester triglycéride de l'acide butyrique - 0,5 à 2 %
glycérol - 45 à 50 %
ou
(b)
ester monoglycéride de l'acide propionique - 45 à 50 %
ester diglycéride de l'acide propionique - 8 à 12 %
ester triglycéride de l'acide propionique - 1 à 3 %
glycérol - 36 à 40 %
ou
(c)
ester monoglycéride de l'acide butyrique - 45 %
ester diglycéride de l'acide butyrique - 6 %
ester triglycéride de l'acide butyrique - 1 %
glycérol - 48 %
ou
(d)
monoglycérides de l'acide butyrique - 43 %
diglycérides de l'acide butyrique - 6 %
triglycérides de l'acide butyrique - 1 %
glycérol - 50 %
ou
(e)
ester monoglycéride de l'acide propionique - 49 %
ester diglycéride de l'acide propionique - 10 %
ester triglycéride de l'acide propionique - 2 %
glycérol - 39 %
ou
(f)
monoglycérides de l'acide propionique - 43 %
diglycérides de l'acide propionique - 6 %
triglycérides de l'acide propionique - 1 %
glycérol - 50 %
lesdits pourcentages étant exprimés en poids.

6. Compositions selon les revendications 1 à 5 contenant également des principes actifs d'huiles essentielles dans des pourcentages compris entre 1 et 20 % (en poids).

7. Utilisation des compositions selon les revendications 1 à 6 en tant que compositions antibactériennes et anti-moisissures pour le traitement de céréales, d'aliments pour animaux et de produits alimentaires et d'eau de boisson en général prévus pour l'alimentation d'animaux.

8. Aliments ou boissons pour animaux, soit liquides soit solides, comprenant une composition contenant des monoglycérides d'acides organiques en C₁ à C₇ dans des pourcentages situés entre 10 % et 90 % en poids calculés sur le poids total de la composition pour une utilisation en tant que compositions antibactériennes et anti-moisissures pour le traitement d'animaux **caractérisés en ce qu'**une telle composition contient du glycérol dans des pourcentages situés entre 10 % et 90 % en poids calculés sur le poids total de la composition.

9. Aliments ou boissons pour animaux selon la revendication 8, dans lesquels lesdites compositions sont ajoutées dans une quantité située entre 0,1 et 1,5 %, de préférence entre 0,3 et 0,5 % en poids sur le poids total de l'aliment ou de la boisson pour animaux.

10. Aliments pour animaux selon les revendications 8 et 9, lesdits aliments pour animaux étant ceux utilisés pour l'alimentation des porcs, des poulets, des poissons, des bovins, des moutons et des animaux de compagnie.
